# EUROPEAN PATENT SPECIFICATION

(11) **EP 1 423 125 B1**
(45) Date of publication and mention of the grant of the patent: **29.08.2007**
(21) Application number: 02761551.7
(22) Date of filing: 03.09.2002
(51) Int. Cl.: A61K 31/56, A61P 27/06

(54) **USE OF NON-FEMINIZING ESTROGENS AS RETINOPROTECTIVE AGENTS FOR THE TREATMENT OF GLAUCOMA**
VERWENDUNG VON NICHT FEMINISIERENDEN ÖSTROGENEN ALS RETINOPROTEKTIVE MITTEL ZUR BEHANDLUNG VON GLAUKOM
OESTROGENES N'INDUISANT PAS DE FEMINISATION UTILISES COMME AGENTS A EFFET PROTECTEUR SUR LA RETINE DANS LE TRAITEMENT DU GLAUCOME

(30) Priority: 05.09.2001 US 317225 P
(43) Date of publication of application: 02.06.2004
(73) Proprietor: Alcon, Inc., 6331 Hünenberg (CH)
(72) Inventor: PANG, Iok-Hou, Grand Prairie, TX 75052 (US); CLARK, Abbot, F., Arlington, TX 76017 (US)
(74) Representative: Teipel, Stephan
(86) International application number: PCT/US2002/027969
(87) International publication number: WO 2003/020284

(56) References cited:
- EP-A- 1 236 469
- WO-A-02/42319
- US-A- 5 521 168
- US-B1- 6 197 833
- M.O.SATOR ET ALL.: "Hormone replacement therapy and intraocular pressure" MATURITAS, vol. 28, 1997, pages 55-58, XP002309890

## Description

### BACKGROUND OF THE INVENTION

### 1. Field of the Invention

The present invention relates to the field of glaucoma. More specifically, the invention provides a method to protect glaucomatous retinopathy using compositions comprising at least one non-feminizing estrogen.

### 2. Description of the Related Art

"Glaucomas" are a group of debilitating eye diseases that are the leading cause of preventable blindness in the United States and other developed nations. Primary Open Angle Glaucoma (POAG) is the most common form of glaucoma. The disease is characterized by the degeneration of the trabecular meshwork (TM), leading to obstruction of the normal ability of aqueous humor to leave the eye without closure of the space (e.g., the "angle") between the iris and cornea (Langham (1979); Segawa (1979); Rohen (1983)). A characteristic of such obstruction in this disease is an increased intraocular pressure (IOP), resulting in progressive visual loss and blindness if not treated appropriately and in a timely fashion. The disease is estimated to affect between 0.4% and 3.3% of all adults over 40 years old (Leske *et al.* (1994, 1997, 2001); Bengtsson (1989); Strong (1992)). Moreover, the prevalence of the disease rises with age to over 6% of those 75 years or older (Strong (1992)).

Another form of POAG, normal-tension glaucoma, is characterized by a severe optic neuropathy in the absence of abnormally high IOP. Patients with normal-tension glaucoma have pressures within the normal range, albeit often in the high normal range (Tanna & Jampel (2000)).

Because increased IOP is a readily measurable characteristic of glaucoma, the diagnosis of the disease is largely based on an increase in IOP which is generally estimated by tonometry (Strong (1992); Greve & Chisholm (1993)). Unfortunately, as is evident from normal-tension glaucoma, glaucomatous retinopathy and optic nerve damage can occur in the absence of abnormally high IOP (Yamamoto & Kitazawa (1998); Tanna & Jampel (2000)). Conversely, ocular hypertension does not always lead directly to retina or optic nerve damage. Approximately 5 million Americans have elevated IOP without optic nerve damage or visual field loss. Because the relationship between pressure and optic nerve and retina damage is not necessarily direct, high IOP is now considered to be only a risk factor rather than an essential disease characteristic. For this reason, additional methods, such as direct examination of the optic disk and determination of the extent of a patient's visual field loss are often conducted to improve the accuracy of diagnosis (Greve & Chisholm (1993)). Also for the same reason, the ultimate goal of glaucoma treatment is to preserve vision by protecting against the pathological changes in the retina and optic nerve.

Current glaucoma therapy is directed to lowering IOP. A variety of therapeutic agents have been proposed as having the ability to reduce elevated IOP. These therapies lower IOP, but they do not directly address the pathogenic mechanisms occurring at the retina and optic nerve, and the disease continues to progress. Moreover, many of these agents are often associated with untoward effects. There is currently no generally accepted therapeutic method to manage glaucomatous retinopathy and optic neuropathy. Agents offering retinoprotection properties would be desirable.

### SUMMARY OF THE INVENTION

The present invention overcomes these and other drawbacks of the prior art by providing compositions comprising at least one non-feminizing estrogen and methods for their use in the treatment of glaucomatous retinopathy. In particular, the invention provides methods for retinoprotection by administering to a patient in need thereof a therapeutically effective amount of a composition including at least one non= feminizing estrogen compound. As used herein, the phrase "non-feminizing estrogen compound" refers to compounds having very little or no feminizing, or sex-related, activity.

It is contemplated that virtually any non-feminizing estrogen compound will be useful in the methods of the invention. Typically, the non-feminizing estrogen compound for use in the methods of the invention will be polycyclic compounds having a terminal phenolic group, in a structure containing at least a second ring, having a molecular mass of less than 1000 Daltons. Examples of such compounds include, estratriene-3-ol, 3,17α-estradiol, estrone, estriol. Most preferably, the non-feminizing estrogen compound is estratriene-3-ol.

### DETAILED DESCRIPTION PREFERRED EMBODIMENTS

Incidence of ocular hypertension and primary open angle glaucoma is known to increase during menopause (Qureshi (1996); Worda & Sator (2000)), which may be related to the sudden decrease of circulating concentration of estrogen in post-menopausal women. Administration of estrogen together with progestin was shown to lower IOP (Meyer *et al.* (1966); Caramazza *et al.* (1968); Treister & Mannor (1970); Sator *et al.* (1998)). Treister and Mannor also observed the IOP-lowering effect with estrogen administration alone (1970). The estrogen induced reduction in IOP correlates with an increase in outflow facility of aqueous humor. However, independent of the IOP-lowering effect, estrogens have been demonstrated to be protective against various insults in the brain (Cyr *et al.* (2000); Emilien *et al.* (2000); Granholm (2000); Green *et al.* (2000); Henderson (1997); McMillan & Dorsa (1999); Monk & Brodaty (2000); Simpkins *et al.* (2000); Woolly (1999)). Estrogen receptor (Kobayashi *et al.* (1998); Ogueta *et al.* (1999); Wickham *et al.* (2000)) and an estrogen-binding protein (Rao (1998)) have been found in the retina. It is likely that estrogen receptor is involved in vision-affecting conditions in the retina. The present inventors contemplate for the first time that estrogens are useful in the prevention, treatment or reduction of retina and optic nerve damages associated with glaucoma independent of their effects on IOP.

Classical estrogens or their metabolites are not practical as therapeutic agents for the treatment of retinal diseases because their feminizing effects are not acceptable to many patients. Non-feminizing estrogen compounds are estrogen-related compounds having substantially no sex-related effect on the subject. Simpkins *et al.* (U.S. Patent No. 6,197,833; U.S. Patent No. 5,877,169; U.S. Patent No. 5,843,934) discuss the use of such compounds for treatment of patients with a number of degenerative conditions or conditions resulting from ischemic damage in the brain. Simpkins *et al.* do not discuss the use of the compounds for the treatment of eye-related diseases. EP 1236469 relates to angiostatic steroids.

Estrogen occurs in at least two isomeric forms, including α estrogen and β estrogen. β estrogens are pleotrophic molecules with many biological activities. Clinical uses include treatment of osteoporosis, symptoms of menopause and fertility control. In comparison to β estrogen, α estrogen is typically believed to be at least 100-1000 times less potent in estrogenic activity. Numerous examples have been reported in the literature that show that the biological effects of β estrogen are not shared by the α isomer. In fact, in the art, α estrogen is typically used as a negative control for β estradiol.

Simpkins *et al.* (U.S. Patent No. 5,843,934) showed the α estrogen has a comparable activity to that of β estrogen for neuroprotection. This activity provides α estrogen with a number of advantages over β estrogen in the treatment of degenerative diseases, trauma and aging related to the central nervous system. These advantages arise in situations which require treatment of males where the development of female traits is to be avoided and the treatment of females where the subject has increased susceptibility to endometrial, breast and cervical cancer. The present inventors show for the first time that non-feminizing estrogens are useful in the treatment of glaucomatous retinopathy.

U.S. Patent No. 5,521,168 discusses the use of estrogen metabolites for lowering of intraocular pressure. The compounds disclosed in this patent are estrogen metabolites, some of which may not have sex-related pharmacological actions. However, U.S. Patent No. 5,521,168 does not discuss the use of these estrogen metabolites or any non-feminizing estrogens in the treatment of glaucoma-related retina and optic nerve damages.

It is contemplated that virtually any non-feminizing estrogen compound will be useful in the methods of the invention. Typically, the non-feminizing estrogen compound for use in the methods of the invention will be a polycyclic compound having a terminal phenolic group, in a structure containing at least a second ring, having a molecular mass of less than 1000 Daltons. Examples of such compounds include, estratriene-3-ol, 3,17α-estradiol, estrone, estriol. Most preferably, the non-feminizing estrogen compound is estratriene-3-ol.

### EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice.

The compositions of the present invention comprise one or more non-feminizing estrogens and a pharmaceutically acceptable vehicle. As used herein, the term "pharmaceutically acceptable vehicle" refers to any formulation Which is acceptable, i.e., safe and provides the appropriate delivery for the desired route of administration, of an effective amount of one or more non-feminizing estrogens. The compositions of the present invention may be administered in a variety of different ways including systemically (e.g., oral administration, intramuscular injection, subcutaneous injection, intravenous injection, U-ansdennal administration and transmucosal administration), topically and by intraocular injection, intraocular perfusion, periocular injection or retrobulbar (sub-tenon) injection.

The exact dosage of the non-feminizing estrogen(s) will vary, but will be determined by skilled clinicians in the art. Various factors affecting the dosage amount include the actual disease to be treated, the severity of condition, the health of the patient, the potency and specific efficacy of the non-feminizing estrogen, and so on. The amount dosed, however, will be in an effective to prevent, treat or ameliorate an ocular disease or disorder, e.g., those described herein; such an amount is referred herein as an "effective amount." In general, the daily dosage of non-feminizing estrogens will range between 0.001 and 100 milligrams per kilogram body weight per day (mg/kg/day), and preferably between about 0.01 and 5.0 mg/kg/day.

The non-feminizitig estrogens of the present invention may be contained in various types of ophthalmic compositions, in accordance with formulation techniques known to those skilled in the art. For example, the compounds may be included in solutions, suspensions and other dosage forms adapted for topical, intravitreal or intracameral use.

Aqueous compositions are generally preferred, based on ease of formulation and physiological compatibility. However, the non-feminizing estrogens of the present invention may also be readily incorporated into other types of compositions, such as suspensions and viscous or semi-viscous gels or other types of solid or semi-solid compositions for topical or retrobulbar injection. The ophthalmic compositions of the present invention may also include various other ingredients, such as buffers, preservatives, co-solvents and viscosity building agents.

An appropriate buffer system (e.g., sodium phosphate, sodium acetate or sodium borate) may be added to prevent pH drift under storage conditions.

Topical ophthalmic products are typically packaged in multi-dose form. Preservatives are thus required to prevent microbial contamination during use. Suitable preservatives include: benzalkonium chloride, chlorobutanol, methyl paraben, propyl paraben, phenylethyl alcohol, edetate disodium, sorbic acid, polyquaternium-1, or other agents known to those skilled in the art. Some of these preservatives, however, may be unsuitable for particular applications, (e.g., benzalkonium chloride may be unsuitable for intraocular injection). Such preservatives are typically employed at a level of from 0.001 to 1.0% weight/volume ("% w/v").

For topical administration of non-feminizing estrogens, the typical dosage generally will range between about 1-2 two drops administered to the eye 1-4 times per day of a composition comprising 0.001 and 5% weight/volume ("w/v"), and preferably between 0.1 and 1% (w/v) of one or more non-feminizing estrogens. Solutions, suspensions, ointments, gels, jellies and other dosage forms adapted for topical administration are preferred. Additionally, non-feminizing estrogens may be delivered slowly, over time, to the afflicted tissue of the eye through the use of contact lenses. This regimen is generally performed by first soaking the lenses in a non-feminizing estrogen solution, and then applying the contact lenses to the eye for normal wear.

The compositions of the present invention are further illustrated in the following formulation examples, non-feminizing estrogens of the present invention are represented generically in the examples as "non-feminizing estrogen."

### Example 1

A topical ophthalmic composition useful for treating retinal vascular diseases:

| **Ingredient** | **Concentration (% w/v)** |
|---|---|
| Non-feminizing estrogen | 0.1 |
| Dibasic Sodium Phosphate | 0.2 |
| HPMC | 0.5 |
| Polysorbate 80 | 0.05 |
| Benzalkonium Chloride | 0.01 |
| Sodium Chloride | 0.75 |
| Edetate Disodium | 0.01 |
| NaOH/HCl | q.s., pH 7.4 |
| Purified Water | q.s. 100% |

### Example 2

A sterile intraocular injection solution useful for treating retinal vascular diseases:

| **Ingredient** | **Concentration (% w/v)** |
|---|---|
| Non-feminizing estrogen | 0.05-5.0 |
| Cremophor EL® | 10 |
| Tromethamine | 0.12 |
| Mannitol | 4.6 |
| Disodium EDTA | 0.1 |
| Hydrochloric acid or sodium hydroxide | q.s., pH to 7.4 |
| Water for injection | q.s. 100% |

### Example 3

A tablet formulation suitable for oral administration, and useful for treating retinal vascular diseases:

| **Ingredient** | **Amount per Tablet (mg)** |
|---|---|
| Non-feminizing estrogen | 200 |
| Cornstarch | 50 |
| Lactose | 145 |
| Magnesium stearate | 5 |

### Example 4

An systemic injectable solution useful for treating retinal vascular diseases:

| **Ingredient** | **Amount** |
|---|---|
| Non-feminizing estrogen | 200 mg |
| 0.4 M KH2PO4 solution | 2 ml |
| 1 N KOH solution | q.s. to pH 7.0 |
| Water for injection | q.s. to 20 ml |

All of the compositions and/or methods disclosed and claimed herein can be made and executed without undue experimentation in light of the present disclosure.

### References

### United States Patents

U.S. Patent No. 5,521,168
U.S. Patent No. 5,843,934
U.S. Patent No. 5,877,169
U.S. Patent No. 6,197,833

### Books

Green and Simpkins "Neuroprotective effectives of phenolic A ring oestrogens," In NEURONAL AND COGNITIVE EFFECTS OF OESTROGENS, Wiley & Sons, new York, pp 202-220 (2000)
Langham ME, "The physiology and pathology of the intraocular pressure, " In GLAUCOMA: CONTEMPORORY INTERNATIONAL CONCEPTS, Ed. Bellows, JG, Masson Publishing, New York, pp 24-48 (1979).
Segawa K, (1979) "Electron microscopic changes in the trabecular tissue in primary open angle glaucoma," In GLAUCOMA: CONTEMPORORY INTERNATIONAL CONCEPTS, Ed. Bellows JG, Masson Publishing, New York, pp 17-23 (1979)
Simpkins JW, Green PS, Gridley KE, Shi J, Monck EK, "Neuroprotective effects of estrogens," In BIOLOGY OF MENOPAUSE, Ed. Bellino FL, Springer-Verlag, New York, pp 103-111 (2000).

### Other Publications

Bengtsson, "Incidence of manifest glaucoma," BR J OPHTHALMOL, vol 73, pp 483-487 (1989)
Caramazza et al, "Le modificazioni indotte da una associazione estrogeno-progestinica sulla dinamica dell'umor acqueo nel glaucoma semplice," ANN OTTAL, vol 94, pp 299-311 (1968)
Cyr et al, "Drugs with estrogen-like potency and brain activity: Potential therapeutic application for the CNS," CURR PHARM DESIGN, vol 6, pp 1287-1312 (2000)
Emilien et al, "Prospects for pharmacological intervention in Alzheimer disease," ARCH NEUROL, vol 57, pp 454-459 (2000)
Granholm, "Oestrogen and nerve growth factor - Neuroptrotection and repair in Alzheimer's disease," EXPERT OPIN INVEST DRUGS, vol 9, pp 685-694 (2000)
Greve and Chisholm, "Comparison of the oculokinetic perimetry glaucoma screener with two types of visual field analyser," CAN J OPHTHALMOL, vol 28, pp 201-6 (1993)
Henderson, "Estrogen replacement therapy for the prevention and treatment of Alzheimer's disease," CNS DRUGS, vol 8, pp 343-351 (1997).
Kobayashi et al, "Estrogen receptor expression in bovine and rat retinas," INVEST OPHTHALMOL Vis SCI, vol 39, pp 2105-2110 (1998).
Leske et al, "The Barbados Eye Study. Prevalence of open angle glaucoma," ARCH OPHTHALMOL, vol 112, pp 821-829 (1994)
Leske et al, "Distribution of intraocular pressure. The Barbados Eye Study," ARCH OPHTHALMOL, vol 115, pp 1051-1057 (1997)
Leske et al, "Incidence of open-angle glaucoma: the Barbados Eye Studies. The Barbados Eye Studies Group," ARCH OPHTHALMOL, vol 119, pp 89-95 (2001)
McMillan and Dorsa, "Estrogen actions in the central nervous system," CURR OPIN ENDOCRINOL DIABETES, vol 6, pp 33-37 (1999)
Meyer et al, "influence of norethynodrel with mestranol on intraocular pressure in glaucoma," ARCH OPHTHALMOL, vol 75, pp 157-161 (1996)
Monk and Brodaty, "Use of estrogens for the prevention and treatment of Alzheimer's disease," DEMENTIA GERIATR COGN DISORD, vol 11, pp 1-10 (2000)
Ogueta SB, Schwartz SD, Yamashita CK, Farber DB, "Estrogen receptor in the human eye: influence of gender and age on gene expression," INVEST OPHTMALMOL VIS SCI, vol 40, pp 1906-1911 (1999)
Qureshi, "Ocular hypertensive effect of menopause with and without systemic hypertension," ACTA OBSTET GYNECOL SCAND, vol 75, pp 266-269 (1996).
Rao, "Isolation and characterization of an estrogen binding protein which may integrate the plethora of estrogenic actions in non-reproductive organs," J STEROID BIOCHEM MOL BIOL, vol 65, pp 3-41 (1998)
Rohen, "Why is intraocular pressure elevated in chronic simple glaucoma? Anatomical considerations," OPHTHALMOLOGY, vol 90, pp 758-765 (1983)
Sator et al, "Reuction of intraocular pressure in a glaucoma patient undergoing hormone replacement therapy," MATURITAS, vol 29, pp 93-95 (1998)
Strong, "How optometrists screen for glaucoma: a survey," OPHTHALMIC PHYSIOL OPT, vol 12, pp 3-7 (1992)
Tanna and Jampel, "Normal-tension glaucoma," OPHTHALMOL CLINICS NORTH AM, vol 13, pp 455-464 (2000)
Treister and Mannor, "Intraocular pressure and outflow facility = effect of estrogen and combined estrogen-progestin treatment in normal human eyes," ARCH OPHTHALMOL, vol 83, pp 311-318 (1970)
Wang et al.; "Estrogen provides neuroprotection in transient forebrain ischemia through perfusion-independent mechanisms in rats, " STROKE, vol 30, pp 630-637 (1999)
Wickham et al., "Identification of androgen, estrogen and progesterone receptor mRNAs in the eye," ACTA OPHTHALMOL SCAND, vol 78, pp 146-153 (2000)
Woolley, "Electrophysiological and cellular effects of estrogen on neuronal function," CRIT REV NEUROBIOL, 13:1-20 (1999)
Worda & Sator, "Hormone replacement therapy and its effect on the eye," J MENOPAUSE, vol 3, pp 24-27 (1999)
Yamamoto and Kitazawa, "Vascular pathogenesis of normal-tension glaucoma: a possible pathogenetic factor, other than intraocular pressure, of glaucomatous optic neuropathy," PROG RETIN EYE RES, vol 17, pp 127-43 (1998)

## Claims

1. Use of a non-feminizing estrogen compound for the manufacture of a medicament for the treatment of glaucoma-related retina or optic nerve damage.

2. The use of claim 1, wherein said glaucoma is primary open angled glaucoma.

3. The use of claim 1, wherein said glaucoma is normal-tension glaucoma (also known as low-tension glaucoma).

4. The use of claim 1, wherein said non-feminizing estrogen compound is a polycyclic compound comprising at least a first ring and a second ring and having a terminal phenolic group wherein said polycyclic compound has a molecular mass of less than 1000 Daltons.

5. The use of claim 1, wherein said non-feminizing estrogen compound is selected from the group consisting of estratriene-3-ol, 3,17α-estradiol, estrone, estriol.

6. The use of claim 1, wherein said medicament is manufactured for administration concurrently or sequentially with one or more IOP-lowering pharmaceutical agents.

7. The use of claim 1, wherein said medicament is manufactured for administration concurrently or sequentially with one or more IOP-lowering surgical procedures.

8. The use of claim 1, wherein said medicament is manufactured for administration concurrently or sequentially with one or more retinoprotective agents.

## Patentansprüche

1. Verwendung einer nicht-feminisierenden Östrogenverbindung zur Herstellung eines Medikamentes für die Behandlung von mit Glaukom in Verbindung stehenden Schädigungen der Netzhaut oder des Sehnervs.

2. Verwendung nach Anspruch 1, wobei das Glaukom in erster Linie ein Weitwinkelglaukom ist.

3. Verwendung nach Anspruch 1, wobei das Glaukom ein Normaldruckglaukom (auch als Niederdruckglaukom bekannt) ist.

4. Verwendung nach Anspruch 1, wobei die nicht-feminisierende Östrogenverbindung eine polycyclische Verbindung ist, die mindestens einen ersten Ring und einen zweiten Ring umfaßt und eine terminale Phenolgruppe ausweist, wobei die polycyclische Verbindung eine molekulare Masse von weniger als 1000 Dalton hat.

5. Verwendung nach Anspruch 1, wobei die nicht-feminisierende Östrogenverbindung aus der Gruppe ausgewählt ist, bestehend aus Östratrien-3-ol, 3,17α-Östradiol, Östron, Östriol.

6. Verwendung nach Anspruch 1, wobei das Medikament für die gleichzeitige oder aufeinanderfolgende Verabreichung mit einem oder mehreren IOP-senkenden Pharmazeutika hergestellt wird.

7. Verwendung nach Anspruch 1, wobei das Medikament für die gleichzeitige oder aufeinanderfolgende Verabreichung mit einem oder mehreren IOP-senkenden Operationsverfahren hergestellt wird.

8. Verwendung nach Anspruch 1, wobei das Medikament für die gleichzeitige oder aufeinanderfolgende Verabreichung mit einem oder mehreren Netzhautschutzmitteln hergestellt wird.

## Revendications

1. Utilisation d'un composé oestrogène non-féminisant pour la fabrication d'un médicament pour le traitement de dommage de la rétine liée à un glaucome ou un endommagement du nerf optique.

2. Utilisation selon la revendication 1, dans laquelle ledit glaucome est un glaucome primaire à angle ouvert.

3. Utilisation selon la revendication 1, dans laquelle ledit glaucome est un glaucome à tension normale (aussi connu en tant que glaucome à basse tension).

4. Utilisation selon la revendication 1, dans laquelle ledit composé oestrogène non-féminisant est un composé polycyclique comprenant au moins un premier cycle et un second cycle et ayant un groupe phénolique terminal dans lequel ledit composé polycyclique a une masse moléculaire inférieure à 1000 Daltons.

5. Utilisation selon la revendication 1, dans laquelle ledit composé oestrogène non-féminisant est choisi parmi le groupe constitué de l'oestratriene-3-ol, du 3,17α-oestradiol, de l'oestrone , de l'oestriol.

6. Utilisation selon la revendication 1, dans laquelle ledit médicament est fabriqué pour une administration de façon simultanée ou séquentielle avec un ou plusieurs agents pharmaceutiques diminuant la pression intra-oculaire.

7. Utilisation selon la revendication 1, dans laquelle ledit médicament est fabriqué pour une administration de façon simultanée ou séquentielle avec une ou plusieurs procédures chirurgicales diminuant la pression intra-oculaire.

8. Utilisation selon la revendication 1, dans laquelle ledit médicament est fabriqué pour une administration de façon simultanée ou séquentielle avec un ou plusieurs agents protecteurs de la rétine.
